# FASCICULE DE BREVET EUROPEEN

(11) **EP 0 813 869 B1**
(45) Date de publication et mention de la délivrance du brevet: **05.03.2003**
(21) Numéro de dépôt: 97401362.5
(22) Date de dépôt: 16.06.1997
(51) Int. Cl.: A61K 31/38, A61K 33/24, A61P 19/02

(54) **Utilisation de sels de strontium pour le traitement de l'arthrose**
Verwendung von Strontiumsälze zur Behandlung von Arthrose
Use of strontium salts for the treatment of arthritis

(30) Priorité: 17.06.1996 FR 9607475
(43) Date de publication de la demande: 29.12.1997
(73) Titulaire: LES LABORATOIRES SERVIER, 92200 Neuilly sur Seine (FR)
(72) Inventeur: Tsouderos, Yannis, 78170 La Celle Saint Cloud (FR); Deloffre, Pascale, 92400 Courbevoie (FR); Wierzbicki, Michel, 78260 L'Etang La Ville (FR)

(56) Documents cités:
- EP-A- 0 415 850
- M. NEUMAN: "Les anti-arthrosiques." ARS MED., vol. 32, no. 24, 1977, pages 2113-2118, XP000645941
- O. SVENSSON ET AL.: "The effect of stontium and manganese on freshly isolated chondrocytes." ACTA PATHOL. MICROBIOL. IMMUNOL. SCAND., vol. 93, no. 3, 1985, pages 115-120, XP000647325
- E. CANALIS ET AL.: "The divalent strontium salt S 12911 enhances bone cell replication and bone formation in vitro." BONE, vol. 18, no. 6, 1996, pages 517-523, XP000646109
- P.J. MARIE ET AL.: "An uncoupling agent containing strontium prevents bone loss by depressing bone resorption and maintaining bone formation in estrogen-deficient rats." J. BONE MINER. RES., vol. 8, no. 5, 1993, pages 607-615, XP000646111

## Description

La présente invention concerne l'utilisation de sels de strontium pour l'obtention de compositions pharmaceutiques destinées à la prévention et au traitement de l'arthrose.

L'utilisation de sels de strontium à usage thérapeutique a déjà fait l'objet de nombreuses publications et de brevets. A titre d'exemple, le brevet US-A-4,152,431 décrit des sels de métaux alcalins utilisables dans le traitement de l'inflammation. Le brevet WO-A-94/09798 présente des complexes de sulfates de différents métaux, actifs dans le traitement des maladies de la peau. Les travaux de Olle Svensson et coll. *(Acta Path. Microbiol. Immunol. Scand.,* Sect. A, *93*, (1985), 115-120) montrent que le strontium joue un rôle dans certains cas de rachitisme.

La demanderesse a présentement découvert que le strontium, sous forme de sels minéraux ou organiques, possède de remarquables propriétés pharmacologiques et trouve une application en thérapeutique tout à fait intéressante dans la prévention et le traitement de l'arthrose.

L'état de l'art est notamment illustré par l'article de M. Neuman (Ars. Med., vol 32, n° 24, 1977).

En effet, il a été découvert que certains sels de strontium stimulent la synthèse, par les chondrocytes humains, des protéoglycanes et du collagène de type II.

Ces molécules sont les deux composants majeurs de la matrice cartilagineuse extracellulaire. C'est la combinaison d'une forte concentration en protéoglycanes incrustés dans un réseau de collagène fibreux qui confère au tissu ses propriétés mécaniques.

Comme la synthèse et la sécrétion de protéoglycanes diminuent avec l'âge, les sels de strontium présentent donc un grand intérêt dans la prévention et le traitement de l'arthrose. Les sels de strontium sont donc particulièrement efficaces pendant le processus de réparation qui intervient dans la phase initiale de la maladie.

La présente invention concerne l'utilisation des sels de strontium bivalent d'acides organiques pour l'obtention de compositions pharmaceutiques destinées à la prévention et au traitement de l'arthrose.

Parmi les acides organiques utilisés pour salifier le strontium afin d'obtenir les sels selon la présente invention, on peut citer plus particulièrement les acides tartrique, malique, maléique, malonique, fumarique, gluconique, oxalique, lactique, succinique, méthanesulfonique, éthanesulfonique, camphorique, citrique ainsi que l'acide 2-[N,N-di(carboxyméthyl)amino]-3-cyano-4-carboxyméthylthiophène-5-carboxylique.

La présente invention concerne l'utilisation selon l'invention du sel de strontium suivant :
Sel distrontique de l'acide 2-[N,N-di(carboxyméthyl)amino]-3-cyano-4-carboxyméthylthiophène-5-carboxylique,

Le sel distrontique de l'acide 2-[N,N-di(carboxyméthyl)amino]-3-cyano-4-carboxyméthylthiophène-5-carboxylique a été décrit dans le brevet EP-B-0 415 850 pour ses propriétés anti-ostéoporotiques et son utilisation dans le traitement du vieillissement cutané et vasculaire, des affections hépatiques et des affections dentaires.

L'arthrose se caractérise anatomiquement par une destruction initiale et primitive des cartilages articulaires.

En conditions normales, le renouvellement du cartilage est un processus très lent consistant en une phase de résorption par les chondrocytes, directement compensée par une phase de formation par ces mêmes chondrocytes.

En conditions pathologiques, le renouvellement du cartilage peut s'accélérer, conduisant à une réaction de réparation précoce du cartilage suivie d'une décompensation cellulaire et d'une dégradation du cartilage. La réaction de réparation résulte d'une multiplication clonale des chondrocytes et de leur synthèse accrue des composants matriciels du cartilage (D. Hamerman et coll., *N. Eng. J. Med.,* (1989), *320* (20), 1322-1330).

Cette réaction homéostatique n'est pas adaptée et dépend d'hormones systémiques et de facteurs de croissance dont les sécrétions diminuent avec l'âge. La résorption du cartilage est régulée par des enzymes et des radicaux libres produits par des tissus adjacents, mais aussi et surtout par le chondrocyte lui-même.

Il est donc tout particulièrement intéressant de disposer de compositions pharmaceutiques permettant d'agir sur le métabolisme chondrocytaire, aussi bien sur la chondroformation que sur la chondrorésorption.

Ces compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Outre un sel de strontium, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple on peut citer :
- *pour les diluants :* le lactose, le dextrose, le sucrose, le mannitol, le sorbitol, la cellulose, la glycérine,
- *pour les lubrifiants :* la silice, le talc, l'acide stéarique et ses sels de magnésium et de calcium, le polyéthylène glycol,
- *pour les liants :* le silicate d'aluminium et de magnésium, l'amidon, la gélatine, la tragacanthe, la méthylcellulose, la carboxyméthylcellulose de sodium et la polyvinylpyrrolidone,
- *pour les désintégrants :* l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 25 mg et 3 g de strontium par 24 heures, par exemple entre 100 mg et 2 g de strontium par 24 heures.

Les exemples suivants illustrent l'invention.

### ETUDE PHARMACOLOGIOUE

### EXEMPLE A : Etudes des effets des sels de strontium selon l'invention sur le métabolisme des chondrocytes

### A) Protocole

### a) Culture de chondrocytes humains

Les chondrocytes sont mis en culture pour une période de courte durée afin de préserver leur phénotype. Dans cette méthode, le cartilage est prélevé sur des genoux de cadavres humains immédiatement après le décès. On pratique une excision des couches superficielles et médianes en évitant la couche calcifiée. La cartilage est découpé en petits fragments puis soumis à digestions enzymatiques. Les fragments de cartilage sont alors successivement traités par la hyaluronidase, la pronase et la collagénase. Premièrement, les morceaux de cartilage sont incubés avec la hyaluronidase, préalablement dissoute dans du Dubbelco's Modified Eagle's medium (DMEM, ICN Biomedical) (0,5 mg/ml ; 3 g de cartilage pour 10 ml de solution enzymatique), pendant 30 minutes à 37°C et sous constante agitation (200 tours par minute).

Deuxièmement, les fragments de cartilage sont placés dans une solution de pronase (1 mg/ml dans le DMEM ; 3 g de cartilage pour 10 ml de solution enzymatique) et incubés avec cette enzyme pendant une heure à 37°C.

Les fragments de cartilage sont ensuite incubés avec la collagénase (1 mg/ml ; 3 g pour 10 ml de solution enzymatique) dissoute dans du DMEM contenant 1% de Ultrosser G (Gibco, Gand, Belgique), pendant 20 heures à 37°C et sous constante agitation (200 tours par minute). Les cellules sont filtrées sur tulle de nylon (diamètre des pores : 25 µm), lavées 3 fois, comptées (nombre variant de 1.10⁶ à 5.10⁶ cellules/ml selon l'étendue de l'étude) puis remises en suspension dans un milieu de culture approprié. Les cellules sont ainsi maintenues en suspension sous agitation constante dans un mélangeur giratoire (100 tours par minute) et sous une atmosphère composée à 95% d'air et à 5% de gaz carbonique. Les cellules et le surnageant sont séparés par centrifugation (1000 tours par minute pendant 5 minutes). Les agrégats de cellules et le surnageant sont conservés à -20°C.

### b) Traitements des cultures chondrocytaires

Les chondrocytes (± 1,5.10⁶ cellules/ml) sont mises en culture pour 24, 48 et 72 heures en absence ou en présence d'un sel de strontium à tester, aux concentrations de 10⁻⁴M, 5.10⁻⁴ M et 10⁻³M. Pour chaque concentration de composé à tester et pour les contrôles correspondants, un lot de trois flacons de culture a été mis en oeuvre. Chaque flacon contient ± 1 à 1,5.10⁶ chondrocytes. L'expérience est répétée deux fois sur deux donneurs différents. Les protéoglycanes et le collagène II sont mesurés dans les surnageants et dans les phases cellulaires.

### c) Paramètres étudiés

### 1. Paramètres de chondroformation

Les cultures sont réalisées dans le DMEM additionné de 1% de ITS+ et d'ascorbate à 50 µg/ml. ITS+ contient : 6,25 µg/ml d'insuline, 625 µg/ml de transferrine, 6,25 µg/ml de sélénium, 1,25 mg/ml d'albumine de sérum bovin et 535 µg d'acide linoléique. Les protéoglycanes et le collagène de type II sont directement dosés dans le surnageant préalablement additionné d'inhibiteurs de protéase. Avant les mesures, les agrégats de cellules sont homogénéisés par dissociation ultrasonique (3 impulsions de 10 secondes) dans du PBS contenant les inhibiteurs de protéase. Les inhibiteurs de protéase utilisés dans cette étude sont l'acide 6-aminohexanoïque (0,1 M), le chlorhydrate de benzamidine (0,05 M), un inhibiteur de trypsine (5.10⁻⁸ M), l'EDTA (0,01 M) et le nitrure de sodium (6,7.10⁻³ M).

### * Dosage radio-immunologique (RIA) des protéoglycanes

Les protéoglycanes libérés dans le milieu de culture (MC) et présents dans les agrégats chondrocytaires (AC) sont dosés selon la méthode décrite par P. Gysen et P. Franchimont (*J. Immunoassay,* (1984), *5*, 221-243). La sensibilité analytique du RIA est de 0,6 ng/tube.

Les coefficients de variation intra- et inter-dosage sont inférieurs à 10 et 20% respectivement, le long de la partie linéaire de la courbe. Les anticorps sont dirigés uniquement sur le déterminant antigénique du noyau de la protéine. La somme des protéoglycanes dans le MC et dans les AC correspond à la production totale de la molécule.

### * Synthèse de glycosaminoglycanes sulfatés

Cette méthode évalue la production de protéoglycanes sulfatés par incorporation de ³⁵SO₄. Les chondrocytes traités par les sels de strontium sont cultivés en présence de Na₂³⁵SO₄ pendant 24 heures. Les cellules sont séparées pour centrifugation et rinçées. Les glycosaminoglycanes présents dans le milieu de culture et les cellules sont extraits en présence d'inhibiteurs de protéases.

La production totale de glycosaminoglycanes sulfatés au cours des 24 heures de culture est évaluée par comptage de la radioactivité. Les extraits sont soumis à une chromatographie sur sépharose CL 2B pour étudier leur structure physicochimique.

### * Dosage radio-immunologique (RIA) du collagène de type II

Le collagène de type II libéré dans le milieu de culture et présent dans la phase cellulaire est dosé selon la méthode RIA décrite par Y. Henrotin et coll. *(J. Immunoassay,* (1990), *11,* 555-578). La limite de détection pour le dosage est de 3 ng/tube. Les coefficients de variation intra- et inter-dosage sont respectivement de 8 et 15%. Les anticorps sont dirigés sur la partie hélicoïdale de la molécule native.

### 2. Paramètres de chondrorésorption

Les cultures sont réalisées dans du DMEM additionné de 1% de TS+ et d'ascorbate à 50 µg/ml. TS+ contient : 625 µg/ml de transferrine, 6,25 µg/ml de sélénium, 1,25 mg/ml d'albumine de sérum bovin et 535 µg d'acide linoléique. Les protéoglycanes et le collagène de type II sont directement dosés dans le surnageant préalablement additionné d'inhibiteurs de protéase. Avant les mesures, les agrégats de cellules sont homogénéisés par dissociation ultrasonique (3 impulsions de 10 secondes) dans du PBS contenant les inhibiteurs de protéase.

### * Dosage de l'activité, de la stromélysine

L'activité de dégradation de caséine est dosée par de la caséine-résorufine marquée provenant de lait de vache (Boehringer Mannheim). Dans cette étude, l'activation de stromélysine latente est produite par addition de APMA (acétate para-aminophénylmercurique, Sigma Chemie, Deinsenhofen, Allemagne) à la concentration finale de 2 mM. Le milieu conditionné des chondrocytes est incubé pendant 4 heures à 37°C. le milieu de culture activé est incubé avec 20 µg de caséine-résorufine marquée pendant 18 heures à 37°C dans une solution tampon titrée (Tris-HCl 0,2 M ; pH = 7,8) contenant du chlorure de calcium à 0,02 M. Après cette incubation, la réaction enzymatique est stoppée par addition d'acide trichloracétique à la concentration finale de 1,6%. L'échantillon est centrifugé à 7000 g pendant 15 minutes. Dans ces conditions, la caséine non clivée est totalement précipitée, alors que plus de 95% de la caséine clivée reste dans le surnageant. Les mesures sont effectuées par fluorescence. La longueur d'onde d'excitation est de 574 nm et la longueur d'onde d'émission est de 584 nm.

La courbe étalon est obtenue par incubation de quantités croissantes de stromélysine purifiée. En parallèle, des échantillons contenant 10 mM d'EDTA ajoutés avant l'incubation sont traités de manière similaire, fournissant des valeurs-témoin qui sont soustraites des valeurs obtenues avec le milieu actif. Les valeurs-témoin n'excèdent jamais 5% de la fluorescence mesurée dans le milieu actif. Tous les dosages sont effectués en double.

### B) Résultats

Les résultats sont exprimés en ratios de protéoglycanes et de collagène de type II libérés dans les milieux de culture et présents dans les phases cellulaires, par µg d'ADN. Les moyennes ± les déviations standards des moyennes sont calculées. Les comparaisons entre les groupes sont réalisées en utilisant le test t de Student non-apparié ; les différences sont considérées comme statistiquement significatives lorsque p < 0,05.

### 1. Paramètres de chondroformation

### * Dosage radio-immunologique (RIA) des protéoglycanes

Les résultats suivants montrent les effets, à différentes concentrations, du sel distrontique de l'acide 2-[N,N-di(carboxyméthyl)amino]-3-cyano-4-carboxyméthylthiophène-5-carboxyli-que sur la production totale (milieu de culture + phase cellulaire) de protéoglycanes après 24, 48 et 72 heures de culture.

| ***Temps de culture*** | ***Concentration*** | ***ng*/*µg ADN **** | ***Test t de Student***** |
|---|---|---|---|
| | 0 (contrôle) | 83,4 ± 4,1 | - |
| 24 heures | 10⁻⁴ M | 79,8 ± 3,5 | NS |
| | 5.10⁻⁴ M | 78,5 ± 3,4 | NS |
| | 10⁻³ M | 89,7 ± 4,1 | NS |
| | 0 (contrôle) | 104 ± 7,65 | - |
| 48 heures | 10⁻⁴ M | 111 ± 15,4 | NS |
| | 5.10⁻⁴ M | 123 ± 3,6 | p < 0,05 |
| | 10⁻³ M | 136 ± 13,8 | p < 0,05 |
| | 0 (contrôle) | 120 ± 8,5 | - |
| 72 heures | 10⁻⁴ M | 155 ± 2,51 | p < 0,05 |
| | 5.10⁻⁴ M | 173 ± 3 | p < 0,05 |
| | 10⁻³ M | 176 ± 11,3 | p < 0,05 |

| | | | |
|---|---|---|---|
| ** moyenne* ± *déviation standard* | | | |
| *** NS = Non Significatif* | | | |

### * Production de glycosaminoglycanes sulfatés

Les résultats confirment par une deuxième méthode les effets du sel distrontique de l'acide 2-[N,N-di(carboxyméthyl)amino]-3-cyano-4-carboxyméthylthiophène-5-carboxylique (composé A) sur la synthèse de protéoglycanes et montrent que le chlorure de strontium présente les mêmes effets, ce qui indique que le strontium est responsable de cette stimulation de la chondroformation par les chondrocytes humains.
Les résultats obtenus après 24 heures de culture sont rassemblés dans le tableau ci-dessous :

| ***Composé*/*Concentration*** | ***c.p.m.* / *10***^{***6***} ***cellules*** | ***Test t de Student*** |
|---|---|---|
| Contrôle | 43871 ± 1124 | - |
| Composé A / 10⁻³M | 52 005 ± 983 | p < 0,05 |
| SrCl₂10⁻³M | 52 839 ± 489 | p < 0,05 |

Les profils chromatographiques obtenus avec les deux sels de strontium et en particulier avec le composé A mettent en évidence une synthèse accrue de glycosaminoglycanes de haut poids moléculaires. Le tableau ci-dessous présente la répartition (%) des protéoglycanes synthétisés en fonction de leur poids moléculaire.

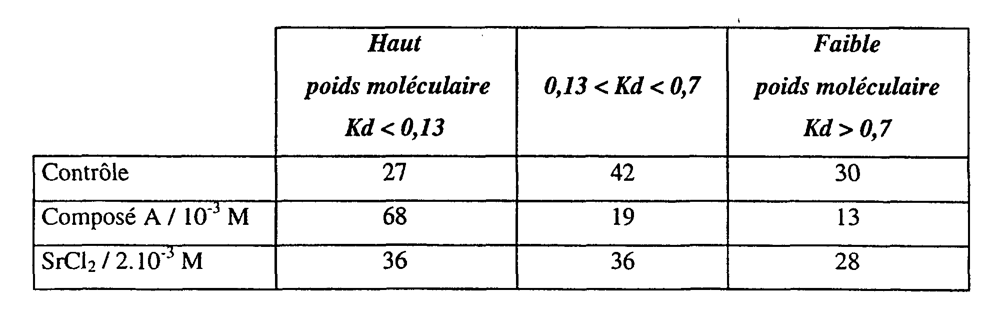

### * Dosage radio-immunologique (RIA) du collagène de type II

Les résultats suivants montrent les effets, à différentes concentrations, du sel distrontique de l'acide (composé A) sur la production de collagène de type II libéré dans le milieu de culture après 24, 48 et 72 heures de culture.

| ***Temps de culture*** | ***Concentration*** | ***n*/*µg ADN**** | ***Test t de Student***** |
|---|---|---|---|
| | 0 (contrôle) | 4,8 ± 0,2 | - |
| 24 heures | 10⁻⁴ M | 5 ± 0,2 | NS |
| | 5.10⁻⁴ M | 5,3 ± 0,3 | NS |
| | 10⁻³ M | 5,1 ± 0,4 | NS |
| | 0 (contrôle) | 6,1 ± 0,5 | - |
| 48 heures | 10⁻⁴ M | 6,6 ± 0,3 | NS |
| | 5.10⁻⁴ M | 7,2 ± 0,5 | NS |
| | 10⁻³ M | 8,6 ± 0,2 | p < 0,05 |
| | 0 (contrôle) | 6,4 ± 0,25 | - |
| 72 heures | 10⁻⁴ M | 5,9 ± 0,8 | NS |
| | 5.10⁻⁴ M | 7,4 ± 0,3 | p < 0,05 |
| | 10⁻³ M | 8,8 ± 0,4 | p < 0,05 |

| | | | |
|---|---|---|---|
| ** moyenne* ± *déviation standard* | | | |
| *** NS = Non Significatif* | | | |

### 2. Paramètres de chondrorésorption

### * Dosage de l'activité de la stromélysine

Les résultats suivants montrent les effets, à différentes concentrations, du sel distrontique de l'acide 2-[N,N-di(carboxyméthyl)amino]-3-cyano-4-carboxyméthylthiophène-5-carboxylique sur la production de stromélysine après 24, 48 et 72 heures de culture.

| ***Temps de culture*** | ***Concentration*** | ***ng*/*µg ADN**** | ***Test t de* Student**** |
|---|---|---|---|
| | 0 (contrôle) | 0,69 ± 0,021 | - |
| 24 heures | 10⁻⁴ M | 0,73 ± 0,072 | NS |
| | 5.10⁻⁴ M | 0,72 ± 0026 | NS |
| | 10⁻³ M | 0,75 ± 0,026 | NS |
| | 0 (contrôle) | 1,43 ± 0,025 | - |
| 48 heures | 10⁻⁴ M | 1,45 ± 0,077 | NS |
| | 5.10⁻⁴ M | 1,48 ± 0,071 | NS |
| | 10⁻³ M | 1,51 ± 0,010 | NS |
| | 0 (contrôle) | 1,79 ± 0,065 | - |
| 72 heures | 10⁻⁴ M | 1,87 ± 0,025 | NS |
| | 5.10⁻⁴M | 1,78 ± 0,15 | NS |
| | 10⁻³M | 2,05 ± 0,16 | p < 0,05 |

| | | | |
|---|---|---|---|
| * *moyenne ± déviation standard* | | | |
| ** *NS = Non Significatif* | | | |

### EXEMPLE B : COMPOSITION PHARMACEUTIQUE : COMPRIMES

Comprimés dosés à 500 mg de sel distrontique de l'acide 2-[N,N-di(carboxyméthyl)amino]-3-cyano-4-carboxyméthylthiophène-5-carboxylique, soit 170 mg de strontium.

| Formule de préparation pour 1000 comprimés : | |
|---|---|
| Sel distrontique de l'acide 2-[N,N-di(carboxyméthyl)amino]-3-cyano-4-carboxyméthylthiophène-5-carboxylique | 500 g (soit 170 mg de strontium) |
| Amidon de blé | 1000 g |
| Lactose | 800 g |
| Stéarate de magnésium | 50 g |
| Silice | 20 g |
| Hydroxypropylcellulose | 50 g |

## Revendications

1. Utilisation du sel distrontique de l'acide 2-[N,N-di(carboxyméthyl)amine]-3-cyano-4-carboxyméthylthiophène-5-carboxylique pour l'obtention de compositions pharmaceutiques destinées à la prévention et au traitement de l'arthrose.

## Claims

1. Use of the distrontium salt of 2-[N,N-di(carboxymethyl)amino]-3-cyano-4-carboxymethylthiophene-5-carboxylic acid in the preparation of pharmaceutical compositions for the prevention and treatment of arthrosis.

## Patentansprüche

1. Verwendung des Distrontiumsalzes der 2-[N,N-Di-(carboxymethyl)-amino]-3-cyano-4-carboxymethylthiophen-5-carbonsäure für die Herstellung von pharmazeutischen Zusammensetzungen zur Vorbeugung oder Behandlung der Arthrose.
